(19) Europäisches Patentamt
European Patent Office
Office européen des brevets

(11) Veröffentlichungsnummer: 0 029 505
B1

(12) # EUROPÄISCHE PATENTSCHRIFT

(45) Veröffentlichungstag der Patentschrift: **12.09.84**

(51) Int. Cl.³: **C 07 D 233/50**, A 61 K 31/415

(21) Anmeldenummer: **80106396.7**

(22) Anmeldetag: **21.10.80**

(54) **Substituierte 2-Phenylamino-imidazoline-(2), deren Säureadditionssalze, diese enthaltende Arzneimittel und Verfahren zur Herstellung derselben.**

(30) Priorität: **26.11.79 DE 2947563**

(43) Veröffentlichungstag der Anmeldung: **03.06.81 Patentblatt 81/22**

(45) Bekanntmachung des Hinweises auf die Patenterteilung: **12.09.84 Patentblatt 84/37**

(84) Benannte Vertragsstaaten: **AT BE CH DE FR GB IT LI LU NL SE**

(56) Entgegenhaltungen:
**EP - A - 0 007 406**
**EP - A - 0 007 422**
**EP - A - 0 007 438**
**DE - A - 1 957 722**
**DE - A - 2 259 160**
**DE - A - 2 523 103**
**DE - A - 2 636 732**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Kobinger, Walter, Prof. Dr., Belghofergasse 27, A-1120 Wien (AT)**
Erfinder: **Lillie, Christian, Dr. Mag., Hansi-Niese-Weg 12, A-1130 Wien (AT)**
Erfinder: **Pichler, Ludwig, Dr., Gusshausstrasse 24, A-1040 Wien (AT)**
Erfinder: **Hoefke, Wolfgang, Dr., Rosselstrasse 21, D-6200 Wiesbaden (DE)**
Erfinder: **Gaida, Wolfram, Dr., Bahnhofstrasse 74, D-6507 Ingelheim (DE)**

**EP 0 029 505 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Substituierte 2-Phenylamino-imidazoline-(2), deren Säureadditionssalze, diese enthaltende Arzneimittel und Verfahren zur Herstellung derselben 2-Phenylamino-imidazoline, die am Phenylaminostickstoff- oder Imidazolin-Ringstickstoffatom substituiert sind, sind z.B. aus DE-A-2 259 160, DE-A-1 957 722, DE-A-2 636 732, DE-A-2 523 103, EP-A-7438, EP-A-7422 und EP-A-7406 bekannt. Verbindungen dieser Art werden vielfältige pharmakologische Wirkungen zugeschrieben. Hervorzuheben sind insbesondere die blütdrucksenkenden, analgetische, sedativen und sekretionshemmenden Eigenschaften. Soweit es sich um vorveröffentlichte Druckschriften handelt, ist in diesen eine bradycarde Wirkung ähnlicher Verbindungen nicht beschrieben.

Es wurde nun gefunden, dass neue Verbindungen vergleichbarer Konstitution überraschenderweise eine gegenüber den bekannten Derivaten des Standes der Technik überlegene bradycarde Wirkung aufweisen.

Die Erfindung betrifft neue substituierte 2-Phenylaminoimidazoline-(2) der allgemeinen Formel (I)

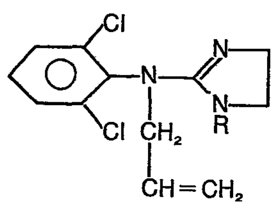

I

sowie deren Säureadditionssalze, insbesondere physiologisch verträgliche Säureadditionssalze, mit wertvollen therapeutischen Eigenschaften.

In der Formel I bedeutet R Methyl oder Ethyl.

Die Herstellung der Verbindungen der Formel I erfolgt durch

(a) Umsetzung eines 2-Phenylamino-imidazolins-(2) der allgemeinen Formel (II)

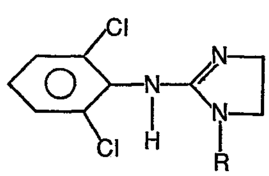

II

in der R die oben genannten Bedeutungen besitzt, mit einem Halogenid der allgemeinen Formel (III)

$$Hal{-}CH_2{-}CH{=}CH_2 \qquad III$$

worin Hal ein Chlor-, Brom- oder Jodatom bedeutet; oder

(b) Umsetzung einer Verbindung der allgemeinen Formel (IV)

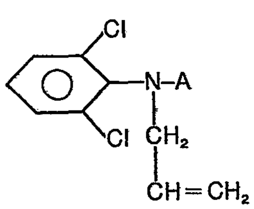

IV

in der A eine Cyanogruppe oder den Rest

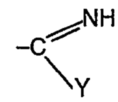

bedeutet, wobei Y eine Alkoxy- oder Alkylthiogruppe mit bis zu 4 C-Atomen oder eine Sulfhydrilgruppe oder eine Aminogruppe darstellt, mit einem Alkylendiamin der allgemeinen Formel (V)

$$H_2N{-}CH_2{-}CH_2{-}NH{-}R \qquad (V)$$

worin R die oben angegebene Bedeutung hat; oder

(c) Umsetzung eines Imidazolinderivates der allgemeinen Formel (VI)

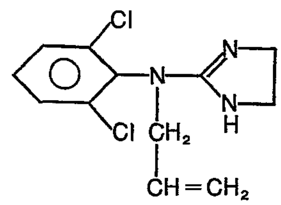

VI

bzw. dessen Anions mit einem Methyl- oder Ethylhalogenid.

Die Umsetzung nach Verfahren (a) erfolgt durch Erhitzen der Reaktionspartner – vorzugsweise in Gegenwart eines polaren oder unpolaren Lösungsmittels – auf Temperaturen von 40 bis 150 °C.

Die speziellen Reaktionsbedingungen hängen in starkem Masse von der Reaktivität der Umsetzungsteilnehmer ab. Es empfiehlt sich, bei der Alkylierung des Halogenid der allgemeinen Formel (III) im Überschuss anzuwenden und die Umsetzung in Gegenwart eines säurebindenden Mittels durchzuführen.

Beim Verfahren (b) ist es erforderlich, bei erhöhter Temperatur, zwischen 60 und 180 °C, zu arbeiten. Lösungsmittel sind nicht notwendig, können aber eingesetzt werden.

Auch bei dem Verfahren (c) kann bei erhöhter Temperatur gearbeitet werden, zweckmässigerweise zwischen 40 und 120 °C. Als Lösungsmittel können polare oder unpolare Solventien dienen. Zweckmässigerweise werden beim Verfahren (c) mittels basischer Stoffe, wie beispielsweise Natriumhydrid, die Imidazolinderivate der allgemeinen Formel VI in die Anionen überführt und diese mit den Methyl- oder Ethylhalogeniden zur Reaktion gebracht. Es bewähren sich hierbei inerte Lösungsmittel wie Tetrahydrofuran, Dioxan, Dibutyläther und dergleichen.

Ausgangsverbindungen der Formeln II, IV, V oder VI sind literaturbekannt (vgl. z.B. BE-A-623 305 oder DE-A-2 457 979 oder Chem. Ber. 107, 2644 (1974) oder Liebigs Ann. Chem. 751, 159 (1971)).

Die erfindungsgemässen 2-Phenylamino-imidazoline-(2) der allgemeinen Formel (I) können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin.

Die neuen Verbindungen der allgemeinen Formel (I) und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie intakten, narkotisierten Ratten untersucht.

Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung.

Herstellungsbeispiele

Beispiel 1

1-Methyl-2-[N-allyl-N-(2,6-dichlor-phenyl)-amino]-2-imidazolin

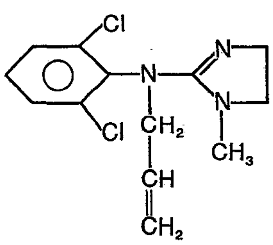

Herstellung nach Verfahren (a)

4,9 g (0,02 Mol) 1-Methyl-2-(2,6-dichlorphenylimino) imidazolidin werden zusammen mit 2,1 ml Allylbromid (125%) und 2,7 g Soda in 25 ml Ethanol 5,5 Stunden unter Rühren am Rückfluss erhitzt. Die Reaktionsmischung wird filtriert und das Filtrat im Vakuum zur Trockne eingeengt. Der verbleibende Rückstand wird in verdünnter, etwa 1 N Salzsäure gelöst und die salzsaure Lösung mit Ether extrahiert (Etherextrakte werden verworfen). Bei aufsteigenden pH-Werten wird sodann fraktioniert mit Ether extrahiert (fraktioniertes Alkalisieren mittels 2 N Natronlauge). Auf diese Weise erhält man etwa 10 Etherfraktionen. Die dünnschichtchromatografisch einheitlichen Extrakte werden vereinigt, über $MgSO_4$ getrocknet und der Ether im Vakuum abgezogen. Im Rückstand verbleibt

1-Methyl-2-[N-allyl-N-(2,6-dichlorphenyl)amino]-2-imidazolin

als Öl, welches nach einiger Zeit durchkristallisiert.

Ausbeute: 2,3 g (entsprechend 40,5% der Theorie)

Schmp.: 59 bis 61 °C.

Herstellung nach Verfahren (c)

2,7 g (0,01 Mol 2-[N-Allyl-N-(2,6-dichlorphenyl)-amino]-2-imidazolin werden zusammen mit 0,44 g 55%iger Natriumhydrid-Dispersion in 25 ml absolutem Tetrahydrofuran 5,5 Stunden unter Rühren am Rückfluss erhitzt. Über Nacht lässt man bei Raumtemperatur stehen und versetzt die Reaktionsmischung mit 1 ml Methyljodid (etwa 160%). Anschliessend wird 2 Stunden am Rückfluss erhitzt. Nach dem Einengen im Vakuum zur Trockene wird der Rückstand in verdünnter, etwa 1 N Salzsäure gelöst und die salzsaure Lösung mehrmals mit Ether extrahiert (Etherextrakte werden verworfen). Bei aufsteigenden pH-Werten wird fraktioniert mit Ether extrahiert (fraktioniertes Alkalisieren mit 2 N NaOH). Die einheitlichen Fraktionen (Kontrolle durch Dünnschichtchromatogramm) werden vereinigt, über $MgSO_4$ getrocknet und im Vakuum das Lösungsmittel abgezogen. Der zunächst ölige Rückstand kristallisiert nach kurzer Zeit durch.

Ausbeute: 0,65 g (entsprechend 23,0% der Theorie)

Schmp.: 59 bis 61 °C.

Die Identität des nach (a) bzw. (c) hergestellten Imidazolins ergab sich durch Dünnschichtchromatogramm, Infrarot-, Kernmagnetisches Resonanz- und Massenspektrum.

Beispiel 2

1-Ethyl-2-[N-(2,6-dichlorphenyl)-N-allyl-amino]-2-imidazolin-hydrobromid

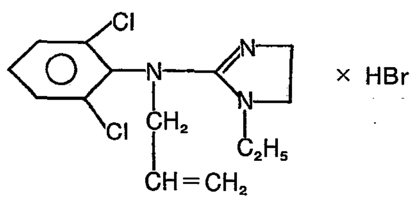

Die Verbindung wurde in analoger Weise wie in Beispiel 1 nach Verfahren (a) aus 1-Ethyl-2-(2,6-dichlorphenylimino)imidazolidin und Allylbromid in einer Ausbeute von 44,2% der Theorie hergestellt.

Schmp.: 167 bis 169 °C.

Formulierungsbeispiele

Beispiel A: Dragées

| | |
|---|---|
| Wirkstoff gemäss Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:

Der Wirkstoff wird mit einem Teil der Hilfsstoffe vermischt, intensiv mit einer wässrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht gepresst, die dann in üblicher Weise mit Hilfe von Zucker, Talkum und Gummi arabicum dragiert werden.

Beispiel B: Ampullen

| | | |
|---|---|---|
| Wirkstoff gemäss Erfindung | | 1,0 mg |
| Natriumchlorid | | 18,0 mg |
| dest. Wasser | ad | 2,0 mg |

Herstellung:

Wirkstoff und Natriumchlorid werden in Wasser gelöst und unter Stickstoff in Glasampullen abgefüllt.

Beispiel C: Tropfen

| | | |
|---|---|---|
| Wirkstoff gemäss Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche für die Vertragsstaaten BE, CH, DE, FR, IT, LI, LU, NL, SE**

1. Substituierte 2-Phenylamino-imidazoline-(2), der allgemeinen Formel (I)

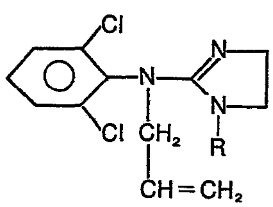
I

worin R Methyl oder Ethyl bedeutet sowie deren Säureadditionssalze.

2. Verfahren zur Herstellung von substituierten 2-Phenylaminoimidazolinen nach Anspruch 1, dadurch gekennzeichnet, dass man

a) ein 2-Phenylamino-imidazolin-(2) der allgemeinen Formel (II)

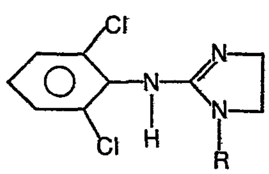
II

in der R die oben genannten Bedeutungen besitzt, mit einem Halogenid der allgemeinen Formel (III)

$$Hal{-}CH_2{-}CH{=}CH_2 \qquad III$$

worin Hal ein Chlor-, Brom- oder Jodatom bedeutet, bei 40 bis 150 °C umsetzt, oder

b) eine Verbindung der allgemeinen Formel (IV)

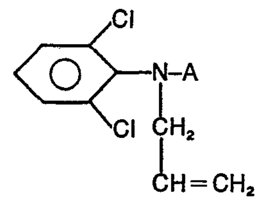
IV

in der A eine Cyanogruppe oder den Rest

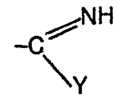

bedeutet, wobei Y eine Alkoxy- oder Alkylthiogruppe mit bis zu 4 C-Atomen oder eine Sulfhydrilgruppe oder eine Aminogruppe darstellt, mit einem Alkylendiamin der allgemeinen Formel (V)

$$H_2N{-}CH_2{-}CH_2{-}NH{-}R \qquad V$$

worin R die oben angegebene Bedeutung hat, bei 60 bis 180 °C umsetzt, oder

c) ein Imidazolinderivat der allgemeinen Formel (VI)

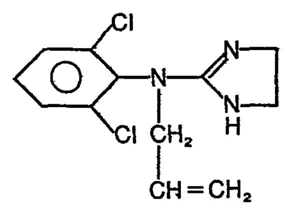
VI

bzw. dessen Anion mit einem Ethyl- oder Methylhalogenid umsetzt

und dass man gegebenenfalls das nach einem Verfahren erhaltene Endprodukt in ein Säureadditionssalz überführt.

3. Pharmazeutische Zubereitungen, dadurch gekennzeichnet, dass sie als Wirkstoff eine oder mehrere Verbindungen nach Anspruch 1 enthalten.

4. Verfahren zur Herstellung von Arzneimitteln nach Anspruch 3, dadurch gekennzeichnet, dass man eine oder mehrere Verbindungen nach Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

5. Verbindungen nach Anspruch 1 zur Verwendung bei der Bekämpfung von Herz- und Coronarerkrankungen.

**Patentansprüche für den Vertragsstaat AT**

1. Verfahren zur Herstellung von substituierten 2-Phenylamino-imidazolinen (2) der allgemeinen Formel (I)

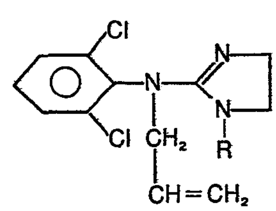
I

worin R Methyl oder Ethyl bedeutet, sowie von

deren Säureadditionssalzen, dadurch gekennzeichnet, dass man

a) ein 2-Phenylamino-imidazolin-(2) der allgemeinen Formel (II)

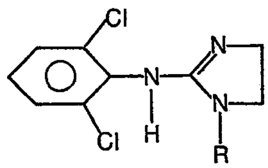

II

in der R die oben genannte Bedeutung besitzt, mit einem Halogenid der allgemeinen Formel (III)

$$Hal{-}CH_2{-}CH{=}CH_2 \qquad III$$

worin Hal ein Chlor-, Brom- oder Jodatom bedeutet, bei 40 bis 150 °C umsetzt, oder

b) eine Verbindung der allgemeinen Formel (IV)

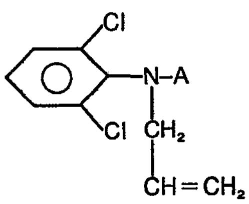

IV

in der

A eine Cyanogruppe oder den Rest

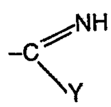

bedeutet, wobei Y eine Alkoxy- oder Alkylthiogruppe mit bis zu 4 C-Atomen oder eine Sulfhydrilgruppe oder eine Aminogruppe darstellt, mit einem Alkylendiamin der allgemeinen Formel (V)

$$H_2N{-}CH_2{-}CH_2{-}NH{-}R \qquad V$$

worin R die oben angegebene Bedeutung hat, bei 60 bis 180 °C umsetzt, oder

c) ein Imidazolinderivat der allgemeinen Formel (VI)

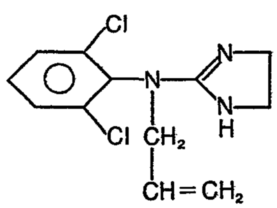

VI

bzw. dessen Anion mit einem Ethyl- oder Methylhalogenid umsetzt

und dass man gegebenenfalls das nach einem Verfahren erhaltene Endprodukt in ein Säureadditionssalz überführt.

2. Verfahren zur Herstellung von Arzneimitteln, dadurch gekennzeichnet, dass man eine oder mehrere Verbindungen der allgemeinen Formel I oder deren Säureadditionssalze nach Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

**Claims for the contracting states BE, CH, DE, FR, IT, LI, LU, NL and SE**

1. Substituted 2-phenylamino-imidazolines (2) of general formula (I)

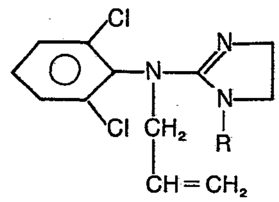

I

in which R represents methyl or ethyl, and their acid addition salts.

2. Process for preparing substituted 2-phenylaminoimidazolines according to claim 1, characterised in that

a) a 2-phenylamino-imidazoline-(2) of general formula (II)

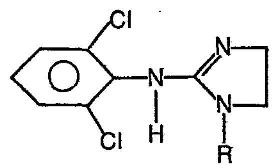

II

in which R has the above-mentioned meanings, is reacted with a halide of general formula (III)

$$Hal{-}CH_2{-}CH{=}CH_2 \qquad III$$

in which Hal represents a chlorine, bromine or iodine atom, at 40 to 150 degrees C or

b) a compound of general formula (IV)

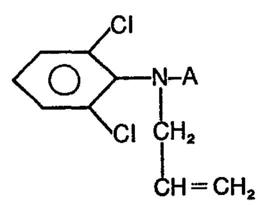

IV

in which A represents a cyano group or the radical

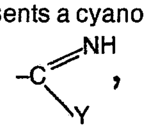 ,

Y representing an alkoxy or alkylthio group with up to 4 carbon atoms or a sulfhydryl group or an amino group, is reacted with an alkyldiamine of general formula (V)

$$H_2N{-}CH_2{-}CH_2{-}NH{-}R \qquad V$$

in which R has the above-mentioned meaning, at 60 to 180 degrees C, or

c) an imidazoline derivative of general formula (VI)

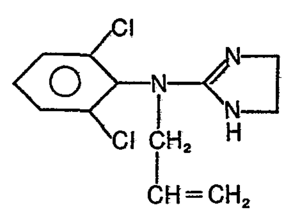

VI

or its anion is reacted with an ethyl or methyl halide, and in that, if appropriate, the final product obtained according to a process is converted into an acid addition salt.

3. Pharmaceutical preparations, characterised in that they contain as active substance one or more compounds according to claim 1.

4. Process for preparing pharmaceuticals according to claim 3, characterised in that one or more compounds according to claim 1 are formulated with conventional galenic excipients, carriers, releasing agents or lubricants or substances for achieving a sustained release effect into tablets, capsules, suppositories, solutions or powders.

5. Compounds according to claim 1 for use in controlling heart and coronary diseases.

**Claims for the contracting state AT**

1. Process for preparing substituted 2-phenylaminoimidazolines-(2) of general formula (I)

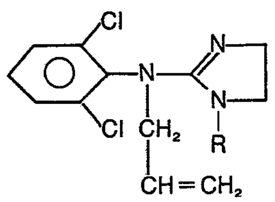

I

in which R represents methyl or ethyl, and their acid addition salts, characterised in that

a) a 2-phenylamino-imidazoline-(2) of general formula (II)

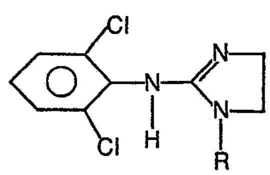

II

in which R has the above-mentioned meanings, is reacted with a halide of general formula (III)

$$Hal{-}CH_2{-}CH{=}CH_2 \qquad III$$

in which Hal represents a chlorine, bromine or iodine atom, at 40 to 150 degrees C, or

b) a compound of general formula (IV)

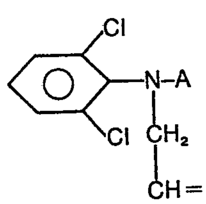

IV

in which A represents a cyano group or the radical

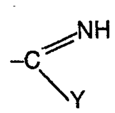

Y representing an alkoxy or alkylthio group with up to 4 carbon atoms or a sulfhydryl group or an amino group, is reacted with an alkylene diamine or general formula (V)

$$H_2N{-}CH_2{-}CH_2{-}NH{-}R \qquad V$$

in which R has the above-mentioned meaning, at 60 to 180 degrees C, or

c) an imidazoline derivative of general formula (VI)

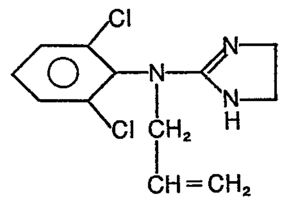

VI

or its anion is reacted with an ethyl or methyl halide, and in that, if appropriate, the final product obtained according to a process is converted into an acid addition salt.

2. Process for preparing pharmaceuticals, characterised in that one or more compounds of general formula (I) or their acid addition salts according to claim 1 are formulated with conventional galenic excipients, carriers, releasing agents or lubricants or substances for achieving a sustained release effect into tablets, capsules, suppositories, solutions or powders.

**Revendications pour les Etats contractants: BE, CH, DE, FR, IT, LI, LU, NL, SE**

1. 2-phénylamino-imidazolines-(2) substituées, de formule générale (I)

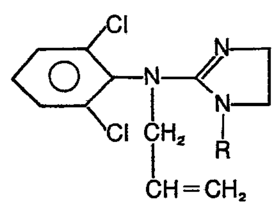

I

dans laquelle R représente méthyle ou éthyle ainsi que leurs sels d'addition d'acides.

2. Procédé pour la préparation de 2-phénylaminoimidazolines substitués selon la revendication 1, caractérisé en ce que

a) on fait réagir une 2-phénylamino-imidazoline-(2) de formule générale (II)

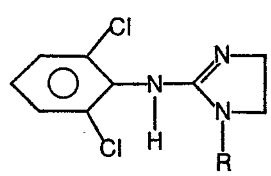

II

dans laquelle R possède les significations mentionnées plus haut, avec un halogénure de formule générale (III)

$$Hal{-}CH_2{-}CH{=}CH_2 \qquad III$$

dans laquelle Hal représente un atome de chlore, de brome ou d'iode, à 40 à 150 °C, ou

b) on fait réagir un composé de formule générale (IV)

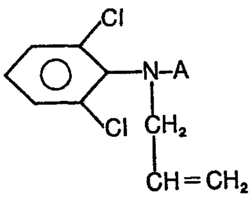

IV

dans laquelle A représente un groupe cyano ou le radical

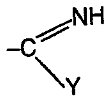

,

Y représentant un groupe alcoxy ou alcoylthio avec jusqu'à 4 atomes de C ou un groupe sulfhydryle ou un groupe amino, avec une alcoylènediamine de formule générale (V)

$$H_2N-CH_2-CH_2-NH-R \quad V$$

dans laquelle R a la signification indiquée plus haut, à 60 à 180 °C, ou

c) on fait réagir un dérivé d'imidazoline de formule générale (VI)

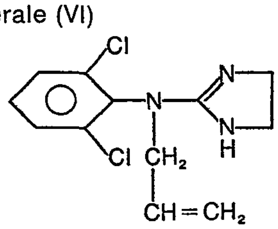

VI

ou respectivement son anion, avec un halogénure d'éthyle ou de méthyle et en ce qu'on transforme éventuellement le produit final obtenu selon l'un des procédés en un sel d'addition d'acide.

3. Préparations pharmaceutiques, caractérisées en ce qu'elles contiennent en tant que substance active un ou plusieurs composés selon la revendication 1.

4. Procédé pour la préparation de médicaments selon la revendication 3, caractérisé en ce que l'on formule un ou plusieurs composés selon la revendication 1 avec des adjuvants, excipients, désagrégeants ou lubrifiants ou respectivement substances pour obtenir un effet retard, galéniques, habituels en comprimés, capsules, suppositoires, solutions ou poudres.

5. Composés selon la revendication 1 pour l'utilisation dans la lutte contre les maladies du cœur et des coronaires.

**Revendications pour l'Etat contractant: AT**

1. Procédé pour la préparation de 2-phénylamino-imidazolines-(2) substituées de formule générale (I)

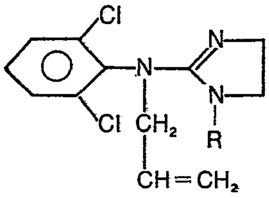

I

dans laquelle R représente méthyle ou éthyle ainsi que de leurs sels d'addition d'acides, caractérisé en ce que

a) on fait réagir une 2-phénylamino-imidazoline-(2) de formule générale (II)

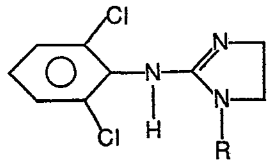

II

dans laquelle R possède les significations mentionnées plus haut, avec un halogénure de formule générale (III)

$$Hal-CH_2-CH=CH_2 \quad III$$

dans laquelle Hal représente un atome de chlore, de brome ou d'iode, à 40 à 150 °C, ou

b) on fait réagir un composé de formule générale (IV)

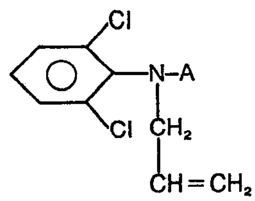

IV

dans laquelle

A représente un groupe cyano ou le radical

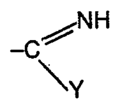

,

Y représentant un groupe alcoxy ou alcoylthio avec jusqu'à 4 atomes de C ou un groupe sulfhydryle ou un groupe amino, avec une alcoylènediamine de formule générale (V)

$$H_2N-CH_2-CH_2-NH-R \quad V$$

dans laquelle R a la signification indiquée plus haut, à 60 à 180 °C, ou

c) on fait réagir un dérivé d'imidazoline de formule générale (VI)

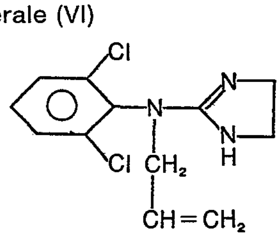

VI

ou respectivement son anion avec un halogénure d'éthyle ou de méthyle et en ce qu'on transforme éventuellement le produit final obtenu selon l'un des procédés en un sel d'addition d'acide.

2. Procédé pour la préparation de médicaments, caractérisé en ce que l'on formule un ou plusieurs composés de formule générale I ou leurs sels d'addition d'acides selon la revendication 1 avec des adjuvants, excipients, désagrégeants ou lubrifiants ou respectivement substances pour obtenir un effet retard, galéniques, habituels en comprimés, capsules, suppositoires, solutions ou poudres.